# EUROPEAN PATENT APPLICATION

(11) **EP 4 692 033 A1**
(43) Date of publication of application: **11.02.2026**
(21) Application number: 24788726.8
(22) Date of filing: 09.04.2024
(51) Int. Cl.: C07C 29/00, C07B 61/00, C07C 31/04

(54) **METHOD FOR PRODUCING LIGNIN DECOMPOSITION PRODUCT, METHOD FOR PRODUCING METHANOL, AND LIGNIN-BASED COMPOSITION**

(30) Priority: 11.04.2023 JP 2023064258
(71) Applicant: Kyushu University, National University Corporation, Nishi-ku Fukuoka-shi Fukuoka 819-0395 (JP)
(72) Inventor: MATSUMOTO, Takahiro, Fukuoka-shi, Fukuoka 819-0395 (JP); UMEMURA, Yuya, Fukuoka-shi, Fukuoka 819-0395 (JP)
(74) Representative: Vossius & Partner Patentanwälte Rechtsanwälte mbB
(86) International application number: PCT/JP2024/014424
(87) International publication number: WO 2024/214702

(57) **Abstract**

A method for producing a lignin decomposition product includes a mixing step of obtaining a mixture comprising a substrate containing lignin and a catalyst containing at least one selected from the group consisting of divalent and trivalent metal ions; and a decomposition step of decomposing the lignin by irradiating the mixture with visible light to obtain a lignin decomposition product. A method for producing methanol includes a step of generating methanol by irradiating, with visible light, a mixture comprising a substrate containing lignin and a catalyst containing at least one selected from the group consisting of divalent and trivalent metal ions.

## Description

### Technical Field

The present disclosure relates to a method for producing a lignin decomposition product, a method for producing methanol, and a lignin-based composition.

### Background Art

In order to realize a carbon-neutral society, methods for obtaining useful chemicals by decomposing lignin, a renewable biomass resource, have been studied. Patent Literature 1 discloses a method for decomposing lignin to obtain various decomposition products by reacting lignin with a transition metal complex catalyst and hydrogen peroxide. Patent Literature 2 describes that an iron ion extract of lignin functions as a cell proliferation inhibitor. Patent Literature 3 discloses a catalyst for lignin decomposition in which a copper compound is fixed on a metal substrate.

### Citation List

### Patent Literature

[Patent Literature 1] PCT International Publication No. 2019/203051
[Patent Literature 2] Japanese Unexamined Patent Publication No. 2020-023583
[Patent Literature 3] PCT International Publication No. 2022/149532

### Summary of Invention

### Technical Problem

Among wood components, cellulose and hemicellulose, which together account for two-thirds, are used as raw materials for pulp. On the other hand, lignin, which accounts for the remaining one-third of wood components, is a rigid and stable substance with a complex chemical structure, and is therefore used as a thermal resource. Therefore, decomposing lignin and converting it into other useful components leads to effective utilization of biomass resources.

Conventionally, in order to decompose lignin having a complex structure, it has been necessary to apply high energy such as high temperature of 100°C or more or high pressure, or to use strong oxidizing or reducing agents such as hydrogen peroxide. In addition, since various low molecular weight chemicals such as methanol, benzene, benzene derivatives, and acetone are produced from lignin decomposed under such harsh reaction conditions, a further step of separating the obtained chemicals has been required. The present disclosure provides a method for producing a lignin decomposition product and a method for producing methanol, which enable decomposition of lignin to obtain a lignin decomposition product by a mild and simple process. The present disclosure also provides a lignin-based composition comprising a lignin decomposition product or a purified product thereof obtained by such a production method.

### Solution to Problem

One aspect of the present disclosure provides a method for producing a lignin decomposition product, the method comprising: a mixing step of obtaining a mixture comprising a substrate containing lignin and a catalyst containing at least one selected from the group consisting of divalent and trivalent metal ions; and a decomposition step of decomposing the lignin by irradiating the mixture with visible light to obtain a lignin decomposition product.

The method for producing a lignin decomposition product can produce a lignin decomposition product by using metal ions as a catalyst and visible light as an energy source. By using such a method, it is possible to decompose lignin and obtain a lignin decomposition product by a mild and simple process.

One aspect of the present disclosure provides a method for producing methanol, the method comprising a step of generating methanol by irradiating, with visible light, a mixture comprising a substrate containing lignin and a catalyst containing at least one selected from the group consisting of divalent and trivalent metal ions.

The method for producing methanol can selectively generate methanol from lignin by using a metal ion catalyst and visible light. In addition, the generation of low molecular weight compounds other than methanol can be reduced, and only methanol can be generated. Therefore, it is not necessary to perform a separation operation between methanol and low molecular weight compounds, and the process can be simplified. Thus, methanol can be obtained by decomposing lignin in a mild and simple process.

One aspect of the present disclosure provides a lignin-based composition comprising a lignin decomposition product obtained by decomposing lignin by irradiating, with visible light, a mixture comprising a substrate containing lignin and a catalyst containing at least one selected from the group consisting of divalent and trivalent metal ions, or a purified product of the lignin decomposition product.

The lignin-based composition includes a lignin decomposition product obtained by decomposing lignin using a catalyst containing metal ions and visible light, or a purified product thereof. Such a lignin-based composition can be obtained by decomposing lignin by a mild and simple process, and can be utilized as a biomass resource with reduced environmental load.

### Advantageous Effects of Invention

The present disclosure provides a method for producing a lignin decomposition product and a method for producing methanol, which enable decomposition of lignin to obtain a lignin decomposition product by a mild and simple process. The present disclosure also provides a lignin-based composition comprising a lignin decomposition product or a purified product thereof obtained by such a production method.

### Brief Description of Drawings

[FIG. 1] (a) is a diagram showing a result of a gas chromatograph of a recovered product obtained by vacuum distillation of a lignin decomposition product in Example 1. (b) is a diagram showing a result of mass spectrometry of (a).
[FIG. 2] (a) is a diagram showing changes in an absorption spectrum in a wavelength range of 250 to 1000 nm when 0 to 6 equivalents of guaiacol are added to iron ions. (b) is a diagram plotting a relationship between absorbance at a wavelength of 470 nm and equivalents of guaiacol.

### Description of Embodiments

Hereinafter, embodiments of the present disclosure will be described. However, the following embodiments are examples for explaining the present disclosure, and are not intended to limit the present disclosure to the following contents. The upper or lower limit values of the numerical ranges specified in this specification may be replaced with any of the values shown in the examples. The upper and lower limit values described individually may be combined arbitrarily. Unless otherwise specified, the materials or components exemplified in this specification may be used alone or in combination with two or more. In the description, the same reference numerals are given to identical elements or elements having identical functions, and redundant description is omitted.

A method for producing a lignin decomposition product according to one embodiment includes a mixing step of obtaining a mixture including a substrate containing lignin and a catalyst containing at least one selected from the group consisting of divalent and trivalent metal ions, and a decomposition step of decomposing the lignin by irradiating the mixture with visible light to obtain a lignin decomposition product. By including such steps, lignin can be decomposed without using high energy such as high temperature or high pressure, or reagents such as strong oxidizing or reducing agents. Therefore, a lignin decomposition product can be obtained by decomposing lignin in a mild and simple process.

The lignin may include at least one selected from the group consisting of kraft lignin and lignin derivative. The lignin may include lignin extracted from bamboo. Kraft lignin may be a dried black liquor removed during production of pulp from wood. The lignin derivative may be obtained by subjecting lignin to some kind of chemical treatment, such as introduction of a functional group or substitution of an atom. Examples of lignin derivatives include lignosulfonate such as sodium lignosulfonate, and modified lignosulfonate such as modified sodium lignosulfonate. From the viewpoint of promoting the reaction, it is preferable that the lignin includes kraft lignin.

The lignin may be used as it is as a substrate, or a biomass resource containing lignin may be used as the substrate. The black liquor may be used as the substrate without drying. The type of biomass resource is not particularly limited. Examples of biomass resources containing lignin include plants such as the genera Cryptomeria, Chamaecyparis, Pinus, Larix, Abies, and Eucalyptus . The biomass resource containing lignin may be bamboo. Any one or more of these may be used in combination. The shape of the substrate may be powder or chip-like. The average particle diameter of the powder may be 1 to 1000 µm, or 100 to 1000 µm. When the average particle diameter of the powder is within the above range, the reaction can be promoted. The average particle diameter can be measured using a laser diffraction/scattering particle size distribution analyzer. For example, "LS-13 320" (product name) manufactured by Beckman Coulter, Inc. can be used as the laser diffraction/scattering particle size distribution measuring apparatus.

The catalyst may comprise at least one selected from the group consisting of iron ions and manganese ions as the metal ion. Such metal ions can be obtained by dissolving a metal compound in water. The catalyst may be present as a complex ion in an aqueous solution. The catalyst may be an ionic metal compound containing iron or manganese as a constituent element. Examples of metal compounds include iron compounds and manganese compounds. Examples of iron compounds include Fe^{III}(NO₃)₃, Fe^{III}Cl₃, Fe^{III}₂(SO₄)₃, Fe^{II}(NO₃)₂, Fe^{II}Cl₂, and Fe^{II}(SO₄), and examples of manganese compounds include Mn^{II}(NO₃)₂. Such a catalyst containing metal ions can be easily prepared because water is used as a solvent. In addition, since the catalyst does not deteriorate after the reaction and can be reused, the decomposition of lignin can be performed more simply, and the environmental load can be reduced. As the metal ion, divalent or trivalent ruthenium ion or osmium ion may also be used. From the viewpoint of further improving the reaction rate, it is preferable that the catalyst contains divalent or trivalent iron ion. By using a metal ion as a catalyst, water can be used as the reaction solvent, and the amounts of reagents such as organic solvents, oxidizing agents, and reducing agents that impose an environmental load can be reduced.

The method for producing a lignin decomposition product includes a mixing step of obtaining a mixture comprising a substrate containing lignin and a catalyst containing at least one selected from the group consisting of divalent and trivalent metal ions. The mixture may contain 0.001 mmol or more, 0.005 mmol or more, 0.007 mmol or more, or 0.01 mmol or more of the catalyst per 100 mg of the substrate containing lignin. This can further promote the reaction. The mixture may contain 10 mmol or less, 5 mmol or less, 1 mmol or less, or 0.5 mmol or less of the catalyst per 100 mg of the substrate containing lignin. The mixture may contain the catalyst in a proportion of 0.001 to 10 mmol, 0.001 to 1 mmol, 0.01 to 0.15 mmol, 0.02 to 0.12 mmol, or 0.03 to 0.10 mmol per 100 mg of the substrate containing lignin. When the content of the catalyst is within the above range, the reaction can be sufficiently promoted. The amount of substrate used per batch is not limited to 100 mg, and may be more than 100 mg or less than 100 mg. The amount of the catalyst can be adjusted within the above range according to the amount of the substrate.

The amount of substrate used per batch may be 1 mg or more, 10 mg or more, 50 mg or more, or 80 mg or more from the viewpoint of increasing the catalyst turnover number and improving the amount of methanol produced. The amount of substrate used per batch may be 1000 mg or less, 500 mg or less, 200 mg or less, or 150 mg or less from the viewpoint of improving the yield. The range of the amount of substrate used per batch may be 1 to 1000 mg, 10 to 500 mg, or 10 to 150 mg.

The method for producing a lignin decomposition product includes a decomposition step of obtaining a lignin decomposition product by irradiating the mixture with visible light. The visible light may include a wavelength of 385 to 740 nm, 400 to 700 nm, or 450 to 600 nm. The light intensity of the visible light may be 10 mW to 500 W, 50 mW to 100 W, 100 mW to 10 W, 150 mW to 1 W, 200 mW to 750 mW, or 250 mW to 500 mW. Such visible light can be easily irradiated and is highly safe. Therefore, a lignin decomposition product can be obtained by a mild and simple process. Such visible light can be irradiated using a visible light irradiation device. For example, "300 W Xenon Light Source MAX-303" (product name) manufactured by Asahi Spectra Co., Ltd. can be used as the visible light irradiation device.

The irradiation of visible light may be performed at room temperature, or may be performed while heating the mixture. The irradiation of visible light may be performed while cooling the mixture. The temperature of the mixture during visible light irradiation may be, for example, 2 to 80°C, 15 to 80°C, or 20 to 60°C. When the temperature of the mixture during visible light irradiation is within the above range, lignin can be decomposed by a mild and simple process to obtain a lignin decomposition product. In addition, the reaction can be promoted by heating the mixture.

In the decomposition step, the mixture may be irradiated with visible light while stirring. Stirring can be performed using a stirrer. By irradiating with visible light while stirring, the reaction can be further promoted. From the viewpoint of increasing the amount of methanol produced, the irradiation time of visible light, i.e., the reaction time, may be 1 hour or more, 10 hours or more, or 15 hours or more. From the viewpoint of energy efficiency required for the reaction, the reaction time may be 168 hours or less, 100 hours or less, or 25 hours or less. The range of irradiation time of visible light, i.e., reaction time, may be 1 to 168 hours, 1 to 100 hours, 1 to 25 hours, 10 to 25 hours, or 15 to 20 hours.

The lignin decomposition product may contain methanol. By the above production method, methanol can be obtained from lignin by a mild and simple process. Methanol can be obtained by isolating methanol from the lignin decomposition product obtained after the decomposition step. The isolation of methanol can be performed by recovering low boiling point compounds by vacuum distillation. The isolation of methanol may also be performed by methods other than vacuum distillation, such as extraction using ion exchange resins or organic solvents. GC-MS or the like can be used for identification and quantification after isolation. The recovered product after vacuum distillation may contain components other than methanol, but may contain only methanol. When the recovered product contains only methanol, no further separation operation is required, and methanol can be obtained by a simplified process.

The lignin decomposition product may contain a catechol structure represented by the following formula (1). Formula (1) contains a catechol structure having two hydroxyl groups at the ortho positions of a benzene ring. By using the above production method, the number of catechol structures in the structure of the lignin decomposition product can be increased, and a lignin decomposition product containing a catechol structure with improved functionality and reactivity can be easily obtained. Such a lignin decomposition product with improved functionality and reactivity can be used as a raw material derived from biomass resources.

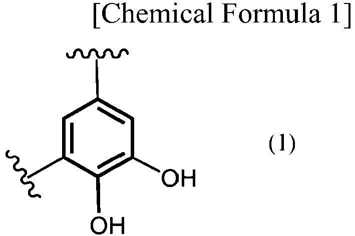

The reaction in which a lignin decomposition product is obtained from lignin and metal ions will be described below with reference to Reaction Scheme (2). Reaction Scheme (2) shows the reaction mechanism when a mixture of lignin and trivalent iron ion is irradiated with visible light. As shown in Reaction Scheme (2), lignin is coordinated to a trivalent iron ion. When light energy from visible light is applied, the methoxy group of lignin and the hydroxyl group of iron ion react and dissociate as methanol. Methanol can be obtained by vacuum distillation of the obtained lignin decomposition product.

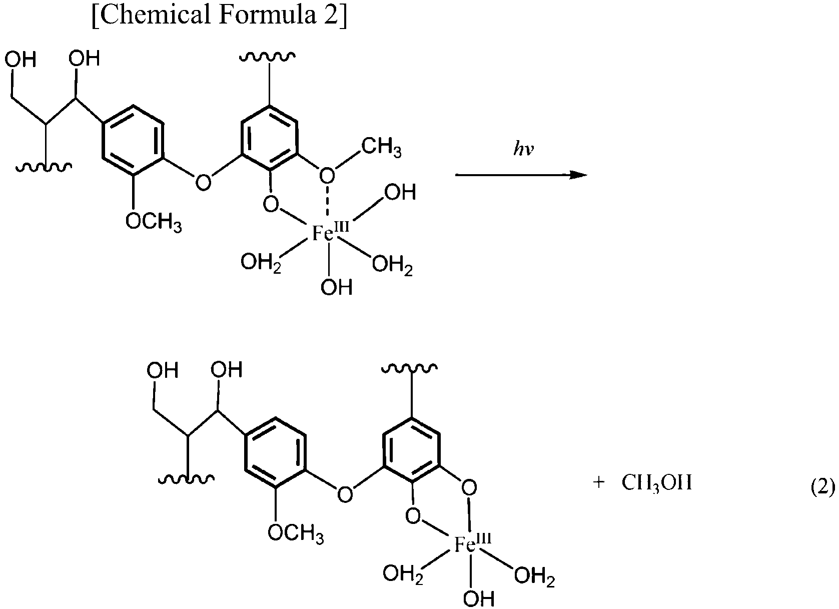

Furthermore, the iron ion coordinated to lignin after the reaction dissociates by the addition of a hydrogen ion to the coordination bond, as shown in Reaction Scheme (3). As a result, a lignin decomposition product containing a catechol structure can be obtained. The hydrogen ion may be derived from water used as the solvent. Since the iron ion can be easily dissociated in this way, the iron ion can be reused as a catalyst. In addition, the residue containing a catechol structure after the reaction can be recovered by filtration or the like. Thus, the catalyst can be reused by a simple process, and the environmental load can be reduced.

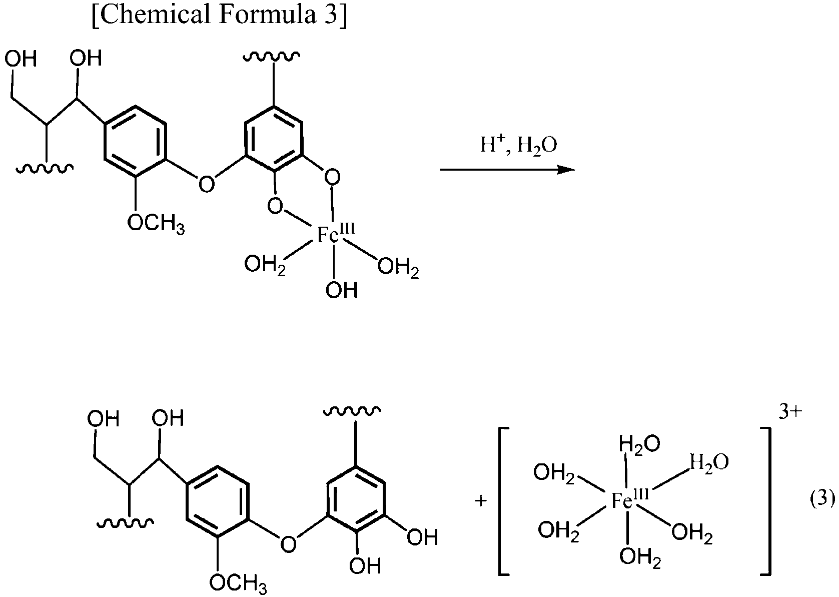

It can be inferred from Reaction Scheme (4) described below that lignin coordinates to an iron ion as shown in Reaction Scheme (2) described above. Reaction Scheme (4) is a scheme showing the coordination of guaiacol to an iron ion when trivalent iron ion and guaiacol are mixed. When 2 equivalents of guaiacol are added to iron ions, the absorbance of the solution increases around a wavelength of 470 nm. Therefore, as shown in Reaction Scheme (4), it is considered that up to two molecules of guaiacol coordinate to an iron ion. Accordingly, it is considered that lignin, which has a structure similar to guaiacol, also coordinates to an iron ion as shown in Reaction Scheme (2).

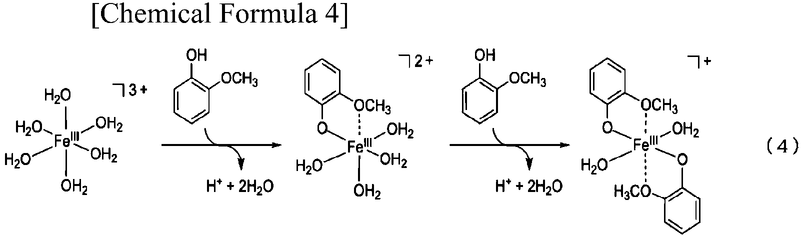

A method for producing methanol according to one embodiment comprises a step of generating methanol by irradiating, with visible light, a mixture including a substrate containing lignin and a catalyst containing at least one selected from the group consisting of divalent and trivalent metal ions. The method for producing methanol can produce methanol by the same procedure as the above-described method for producing a lignin decomposition product. Therefore, the description of the above-described method for producing a lignin decomposition product is also applicable to the method for producing methanol. Thus, methanol can be produced by a mild and simple process.

A lignin-based composition according to one embodiment comprises a lignin decomposition product obtained by decomposing lignin by irradiating, with visible light, a mixture comprising a substrate containing the lignin and a catalyst containing at least one selected from the group consisting of divalent and trivalent metal ions, or a purified product of the lignin decomposition product. The lignin decomposition product can be obtained by the same procedure as the above-described method for producing a lignin decomposition product. Therefore, the description of the above-described method for producing a lignin decomposition product is also applicable to the lignin-based composition. Examples of the purified product of the lignin decomposition product include methanol obtained from the lignin decomposition product, and residue obtained by distilling off light fractions such as methanol from the lignin decomposition product. The residue may contain a catechol structure. The lignin-based composition may contain other components. The lignin-based composition comprising a lignin decomposition product or a purified product thereof obtained by such a production method can be produced by a mild and simple process and can be utilized as a biomass resource with low environmental load.

The present disclosure has been described above with reference to several embodiments, but the present disclosure is not limited to the above embodiments.

The present disclosure includes the following embodiments.
[1] A method for producing a lignin decomposition product, the method comprising:
   a mixing step of obtaining a mixture comprising a substrate containing lignin and a catalyst containing at least one selected from the group consisting of divalent and trivalent metal ions; and
   a decomposition step of decomposing the lignin by irradiating the mixture with visible light to obtain a lignin decomposition product.
[2] The method for producing a lignin decomposition product according to [1], wherein the lignin comprises at least one selected from the group consisting of kraft lignin and lignin derivative.
[3] The method for producing a lignin decomposition product according to [1] or [2], wherein the lignin comprises lignin extracted from bamboo.
[4] The method for producing a lignin decomposition product according to any one of [1] to [3], wherein the visible light includes a wavelength of 385 to 740 nm.
[5] The method for producing a lignin decomposition product according to any one of [1] to [4], wherein the decomposition step comprises irradiating the mixture with the visible light while stirring the mixture.
[6] The method for producing a lignin decomposition product according to any one of [1] to [5], wherein the lignin decomposition product contains methanol.
[7] The method for producing a lignin decomposition product according to any one of [1] to [6], wherein the lignin decomposition product contains a catechol structure represented by the following formula (1). 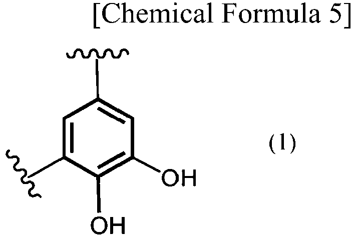
[8] A method for producing methanol, the method comprising a step of generating methanol by irradiating, with visible light, a mixture comprising a substrate containing lignin and a catalyst containing at least one selected from the group consisting of divalent and trivalent metal ions.
[9] A lignin-based composition comprising:
   a lignin decomposition product obtained by decomposing lignin by irradiating, with visible light, a mixture comprising a substrate containing the lignin and a catalyst comprising at least one selected from the group consisting of divalent and trivalent metal ions; or
   a purified product of the lignin decomposition product.
[10] The lignin-based composition according to [9], wherein the lignin decomposition product contains a catechol structure represented by the following formula (1).

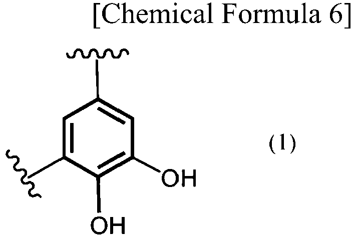

### Examples

Hereinafter, the present disclosure will be described more specifically with reference to Examples and Comparative Examples. However, the present disclosure is not limited to the following Examples.

### (Example 1)

100 mg of kraft lignin (manufactured by Nippon Paper Industries Co., Ltd.) was prepared as a substrate, and an iron compound shown in Table 1 (manufactured by FUJIFILM Wako Pure Chemical Corporation) was prepared as a catalyst. The catalyst was dissolved in 3 g of water so that the amount of catalyst per 100 mg of substrate was 0.04 mmol, and a catalyst aqueous solution was prepared. 100 mg of the substrate was added to the prepared catalyst aqueous solution to prepare a test sample. The test sample was gently stirred at room temperature (about 20°C) using a stirrer, and the reaction between the substrate and the catalyst was started by irradiating with visible light having a wavelength of 385 to 740 nm and a light intensity of 200 mW. A visible light irradiation device (product name: 300 W Xenon Light Source MAX-303, manufactured by Asahi Spectra Co., Ltd.) was used for the visible light irradiation. The reaction temperature was about 20°C, and the irradiation time, i.e., the reaction time, was 18 hours. Other reaction conditions are shown in Table 1.

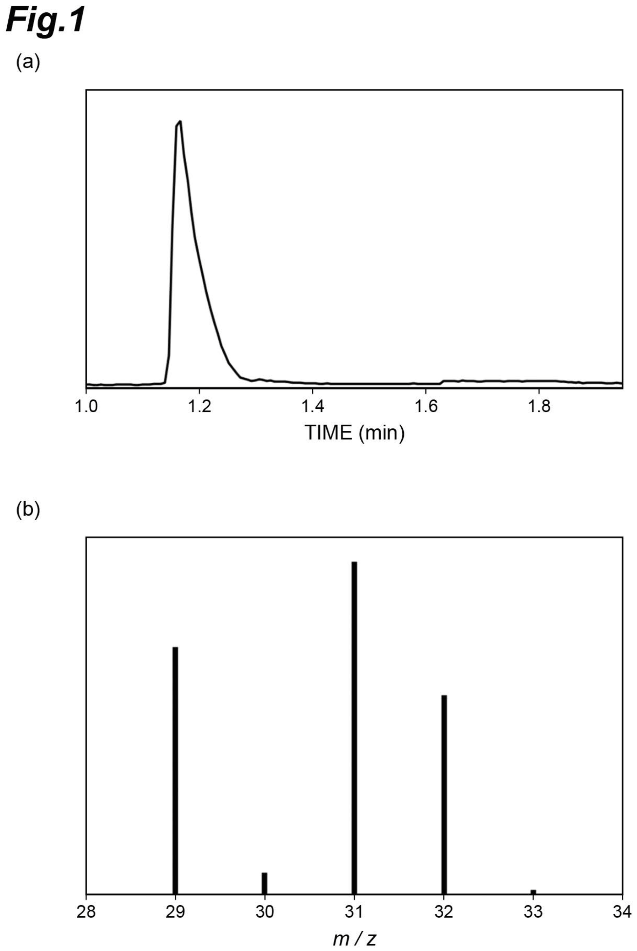

After the reaction, the test sample was subjected to vacuum distillation, and the low boiling point compounds separated from the test sample were measured by GC-MS to qualitatively and quantitatively determine methanol. The peak area of the obtained methanol was converted using a previously prepared calibration curve, and the amount of methanol produced was calculated. The amount of methanol produced is shown in Table 1. The GC-MS measurement results are shown in FIG. 1(a) and FIG. 1(b).

### (Example 2)

Except that the iron compound shown in Table 1 (manufactured by FUJIFILM Wako Pure Chemical Corporation) was used as the catalyst, the reaction was carried out in the same manner as in Example 1, and the amount of methanol produced was measured. The results are shown in Table 1.

### (Example 3)

Except that the iron compound shown in Table 1 (manufactured by FUJIFILM Wako Pure Chemical Corporation) was used as the catalyst, the reaction was carried out in the same manner as in Example 1, and the amount of methanol produced was measured. The results are shown in Table 1.

### (Example 4)

Except that the iron compound shown in Table 1 was used as the catalyst and the reaction was carried out with 0.02 mmol of the catalyst per 100 mg of the substrate, the reaction was carried out in the same manner as in Example 1, and the amount of methanol produced was measured. The results are shown in Table 1.

### (Example 5)

Except that the iron compound shown in Table 1 was used as the catalyst and the reaction was carried out with 0.10 mmol of the catalyst per 100 mg of the substrate, the reaction was carried out in the same manner as in Example 1, and the amount of methanol produced was measured. The results are shown in Table 1.

### (Example 6)

Except that the iron compound shown in Table 1 was used as the catalyst, the amount of substrate used was 50 mg, and the reaction was carried out with 0.04 mmol of the catalyst per 50 mg of the substrate, the reaction was carried out in the same manner as in Example 1, and the amount of methanol produced was measured. The results are shown in Table 1.

### (Example 7)

Except that sodium lignin sulfonate (manufactured by Sigma-Aldrich Co., LLC) was used as the substrate and the iron compound shown in Table 1 was used as the catalyst, the reaction was carried out in the same manner as in Example 1, and the amount of methanol produced was measured. The results are shown in Table 1.

### (Example 8)

Except that commercially available cedar wood powder was used as the substrate, the iron compound shown in Table 1 was used as the catalyst, and the amount of substrate used was 50 mg, the reaction was carried out in the same manner as in Example 1, and the amount of methanol produced was measured. The results are shown in Table 1.

### (Example 9)

Except that wood powder recovered from waste was used as the substrate and the iron compound shown in Table 1 was used as the catalyst, the reaction was carried out in the same manner as in Example 1, and the amount of methanol produced was measured. The results are shown in Table 1.

### (Example 10)

Except that the manganese compound shown in Table 1 (manufactured by Sigma-Aldrich Co., LLC) was used as the catalyst, the reaction was carried out in the same manner as in Example 1, and the amount of methanol produced was measured. The results are shown in Table 1.

### (Comparative Example 1)

Except that cellulose (manufactured by FUJIFILM Wako Pure Chemical Corporation) was used as the substrate and the iron compound shown in Table 1 was used as the catalyst, the reaction was carried out in the same manner as in Example 1, and the amount of methanol produced was measured. The results are shown in Table 1.

### (Comparative Example 2)

A test sample was prepared according to the procedure of Example 1. Thereafter, only stirring was performed for 18 hours without irradiation with visible light, and after stirring, the amount of methanol produced was measured according to the procedure of Example 1. As a result, no methanol was produced in Comparative Example 2.

**[Table 1]**

| | Substrate | Amount of substrate (mg) | Catalyst | Amount of catalyst (mmol) | Amount of produced methanol (mM) |
|---|---|---|---|---|---|
| Example 1 | kraft lignin | 100 | Fe^{III}(NO₃)₃·9H₂O | 0.04 | 1.74 |
| Example 2 | | | Fe^{III} Cl₃·6H₂O | 0.04 | 1.85 |
| Example 3 | | | Fe^{III}₂(SO₄)₃·nH₂O | 0.04 | 4.16 |
| Example 4 | | | | 0.02 | 2.74 |
| Example 5 | | | | 0.10 | 7.81 |
| Example 6 | | 50 | | 0.04 | 5.35 |
| Example 7 | sodium lignin sulfonate | 100 | Fe^{III}₂(SO₄)₃·nH₂O | 0.04 | 1.38 |
| Example 8 | cedar wood powder | 100 | Fe^{III}Cl₃·6H₂O | 0.04 | 0.138 |
| Example 9 | wood powder | 100 | Fe^{III}₂(SO₄)₃·nH₂O | 0.04 | 0.363 |
| Example 10 | kraft lignin | 100 | Mn^{II}(NO₃)₂·nH₂O | 0.04 | 1.08 |
| Comparative Example 1 | cellulose | 100 | Fe^{III}₂(SO₄)₃·nH₂O | 0.04 | 0 |

As shown in FIG. 1(a) and FIG. 1(b), only one peak was observed in the recovered product after vacuum distillation in the gas chromatography result of Example 1, indicating that methanol was selectively produced. As shown in Table 1, when kraft lignin was used as the substrate, Fe^{III}₂(SO₄)₃·nH₂O was used as the catalyst, and the amount of catalyst was 0.10 mmol under visible light irradiation, the amount of methanol produced was the highest. In addition, even when the metal ion contained in the catalyst was divalent manganese ion, methanol was produced. On the other hand, when cellulose was used as the substrate, methanol was not produced. In addition, when cedar wood powder or wood powder containing components other than lignin was used as the substrate, the amount of methanol produced was lower than when kraft lignin was used as the substrate. Therefore, it was found that the metal ions selectively react with lignin as the substrate.

When sodium lignosulfonate was used as the substrate, the amount of methanol produced was less than when kraft lignin was used. Sodium lignosulfonate is a sodium salt in which sulfonic groups are randomly added to lignin. Therefore, it is considered that the sulfonic groups block the portion of lignin that coordinates to iron ions, thereby suppressing the progress of the reaction.

### [Confirmation of Coordination Structure between Guaiacol and Iron Ion]

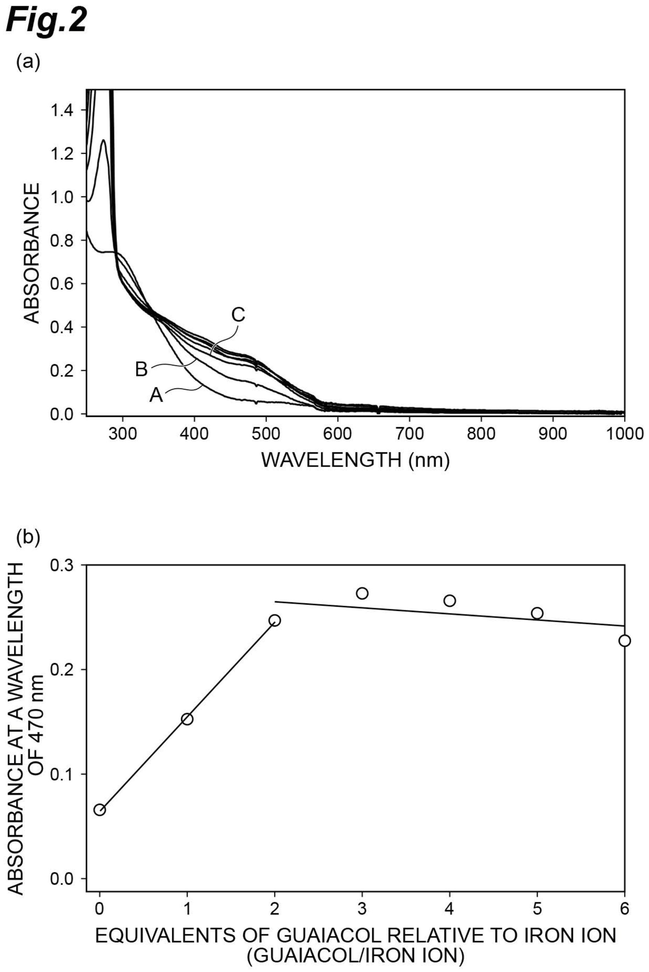

1 to 6 equivalents of guaiacol (manufactured by Tokyo Chemical Industry Co., Ltd.) were added to an aqueous solution of Fe^{III}Cl₃, and the absorption spectrum in the range of 250 to 1000 nm at each equivalent was measured. The results are shown in FIG. 2(a). In FIG. 2(a), A indicates 0 equivalent, B indicates 1 equivalent, C indicates 2 equivalents, and the other spectra are measurement results for 3 equivalents or more. As shown in FIG. 2(a), when guaiacol was added to iron ions, a change in the absorption spectrum was observed around 470 nm. Focusing on the absorbance at a wavelength of 470 nm, as shown in FIG. 2(b), the absorbance increased up to 2 equivalents of guaiacol, and then gradually decreased after 3 equivalents. Therefore, it was confirmed that guaiacol can coordinate up to 2 equivalents to an iron ion. From this, it is considered that lignin, which has a structure similar to guaiacol, also coordinates to metal ions. When light energy from visible light is applied, the metal ions function as a catalyst, and a lignin decomposition product containing methanol can be obtained.

### (Example 11)

110 mg of kraft lignin (manufactured by Nippon Paper Industries Co., Ltd.) was prepared as a substrate, and an iron compound shown in Table 2 (manufactured by FUJIFILM Wako Pure Chemical Corporation) was prepared as a catalyst. The catalyst was dissolved in 3 g of water so that the amount of catalyst per 110 mg of substrate was 0.64 mmol, and a catalyst aqueous solution was prepared. 110 mg of the substrate was added to the prepared catalyst aqueous solution to prepare a test sample. The test sample was cooled to 5°C. Thereafter, the test sample was gently stirred using a stirrer, and the reaction between the substrate and the catalyst was started by irradiating with visible light using the same visible light irradiation device as in Example 1. As shown in Table 2, the reaction temperature was 5°C, and the irradiation time, i.e., the reaction time, was 18 hours. Other reaction conditions are also shown in Table 2. After the reaction, the amount of methanol produced was calculated in the same manner as in Example 1. The results are shown in Table 2.

### (Example 12)

Except that the amount of substrate used was 100 mg, the reaction was carried out with 0.50 mmol of the catalyst per 100 mg of the substrate, and the reaction temperature was room temperature (about 20°C), the reaction was carried out in the same manner as in Example 11, and the amount of methanol produced was measured. The results are shown in Table 2.

### (Example 13)

Except that the amount of substrate used was 10 mg, the reaction was carried out with 0.50 mmol of the catalyst per 10 mg of the substrate, and the reaction temperature was room temperature (about 20°C), the reaction was carried out in the same manner as in Example 11, and the amount of methanol produced was measured. The results are shown in Table 2.

### (Example 14)

Except that the amount of substrate used was 50 mg, the reaction was carried out with 0.04 mmol of the catalyst per 50 mg of the substrate, the reaction temperature was room temperature (about 20°C), and the reaction time was 3 hours, the reaction was carried out in the same manner as in Example 11, and the amount of methanol produced was measured. The results are shown in Table 2.

### (Example 15)

Except that the amount of substrate used was 100 mg, the reaction was carried out with 0.04 mmol of the catalyst per 100 mg of the substrate, the test sample was heated to a reaction temperature of 50°C, and the reaction time was 3 hours, the reaction was carried out in the same manner as in Example 11, and the amount of methanol produced was measured. The results are shown in Table 2.

### (Example 16)

Except that the amount of substrate used was 10 mg, the reaction was carried out with 0.04 mmol of the catalyst per 10 mg of the substrate, the reaction temperature was room temperature (about 20°C), and the reaction time was 3 hours, the reaction was carried out in the same manner as in Example 11, and the amount of methanol produced was measured. The results are shown in Table 2.

### (Example 17)

Except that the amount of substrate used was 10 mg, the reaction was carried out with 0.04 mmol of the catalyst per 10 mg of the substrate, the reaction temperature was room temperature (about 20°C), and the reaction time was 1 hour, the reaction was carried out in the same manner as in Example 11, and the amount of methanol produced was measured. The results are shown in Table 2.

### (Example 18)

Except that the amount of substrate used was 10 mg, the reaction was carried out with 0.08 mmol of the catalyst per 10 mg of the substrate, the reaction temperature was room temperature (about 20°C), and the reaction time was 1 hour, the reaction was carried out in the same manner as in Example 11, and the amount of methanol produced was measured. The results are shown in Table 2.

### (Example 19)

Except that the iron compound shown in Table 2 (manufactured by FUJIFILM Wako Pure Chemical Corporation) was used as the catalyst, the amount of substrate used was 10 mg, the reaction was carried out with 0.04 mmol of the catalyst per 10 mg of the substrate, the reaction temperature was room temperature (about 20°C), and the reaction time was 3 hours, the reaction was carried out in the same manner as in Example 11, and the amount of methanol produced was measured. The results are shown in Table 2.

### (Example 20)

1 mL (1.06 g) of black liquor obtained from acacia (manufactured by Oji Holdings Corporation) was prepared as a substrate. 2 mL of water was added thereto. Furthermore, the iron compound shown in Table 2 was added as a catalyst at a ratio of 0.04 mmol per 1 mL of substrate to prepare a test sample. The test sample was gently stirred at room temperature using a stirrer, and the reaction between the substrate and the catalyst was started by irradiating with visible light using the same visible light irradiation device as in Example 1. The irradiation time, i.e., the reaction time, was 1 hour. The reaction conditions are shown in Table 2. After the reaction, the amount of methanol produced was calculated in the same manner as in Example 1. The results are shown in Table 2.

### (Example 21)

Except that the amount of substrate (black liquor) used was 0.1 mL (0.106 g), 2.9 mL of water was added thereto, and the catalyst was added at a ratio of 0.04 mmol per 0.1 mL of substrate to prepare a test sample, the reaction was carried out in the same manner as in Example 20, and the amount of methanol produced was measured. The results are shown in Table 2.

### (Example 22)

Except that lignin extracted from bamboo was used as the substrate, the iron compound shown in Table 2 was used as the catalyst, the amount of substrate used was 10 mg, the reaction was carried out with 0.04 mmol of the catalyst per 10 mg of the substrate, the reaction temperature was room temperature (about 20°C), and the reaction time was 1 hour, the reaction was carried out in the same manner as in Example 11, and the amount of methanol produced was measured. The results are shown in Table 2. The lignin extracted from bamboo was provided by National University Corporation Oita University.

### (Example 23)

Except that the iron compound shown in Table 2 was used as the catalyst, the amount of substrate used was 10 mg, and the reaction was carried out with 0.80 mmol of the catalyst per 10 mg of the substrate, the reaction was carried out in the same manner as in Example 22, and the amount of methanol produced was measured. The results are shown in Table 2.

### (Example 24)

Except that bamboo itself was used as the substrate, the reaction was carried out in the same manner as in Example 23, and the amount of methanol produced was measured. The results are shown in Table 2. The bamboo was provided by National University Corporation Oita University.

**[Table 2]**

| | Substrate | Amount of substrate | Catalyst | Amount of catalyst (mmol) | Reaction Time (h) | Reaction temperature (°C) | Amount of produced methanol (mM) |
|---|---|---|---|---|---|---|---|
| Example 11 | | 110 mg | Fe^{III}₂(SO₄)₃ ·nH₂O | 0.64 | 18 | 5 | 5.62 |
| Example 12 | | 100 mg | | 0.50 | 18 | room temperature | 15.9 |
| Example 13 | | 10 mg | | 0.50 | 18 | | 5.04 |
| Example 14 | | 50 mg | | 0.04 | 3 | | 2.61 |
| Example 15 | kraft lignin | 100 mg | | 0.04 | 3 | 50 | 5.15 |
| Example 16 | | 10 mg | | 0.04 | 3 | | 1.33 |
| Example 17 | | | | 0.04 | 1 | | 0.92 |
| Example 18 | | | | 0.08 | 1 | | 1.33 |
| Example 19 | | | Fe^{III}Cl₃ ·6H₂O | 0.04 | 3 | room temperature | 0.92 |
| Example 20 | black liquor | 1 mL | Fe^{III}₂(SO₄)₃ ·nH₂O | 0.04 | 1 | | 2.50 |
| Example 21 | | 0.1 mL | | 0.04 | 1 | | 0.64 |
| Example 22 | lignin | 10 mg | | 0.04 | 1 | | 0.11 |
| Example 23 | | | Fe^{III}Cl₃ ·6H₂O | 0.80 | 1 | | 0.21 |
| Example 24 | bamboo | | | 0.80 | 1 | | 0.25 |

As shown in Table 2, a trend was observed in which the amount of methanol produced increased as the amount of substrate increased. A trend was also observed in which the amount of methanol produced increased as the reaction time increased. A trend was also observed in which the amount of methanol produced increased as the reaction temperature increased. Furthermore, a trend was observed in which the amount of methanol produced increased as the amount of catalyst increased.

### Industrial Applicability

According to the present disclosure, it is possible to provide a method for producing a lignin decomposition product and a method for producing methanol, which enable the decomposition of lignin to obtain a lignin decomposition product by a mild and simple process. It is also possible to provide a lignin-based composition comprising a lignin decomposition product or a purified product thereof obtained by such a production method.

## Claims

1. A method for producing a lignin decomposition product, the method comprising:
a mixing step of obtaining a mixture comprising a substrate containing lignin and a catalyst containing at least one selected from the group consisting of divalent and trivalent metal ions; and
a decomposition step of decomposing the lignin by irradiating the mixture with visible light to obtain a lignin decomposition product.

2. The method for producing a lignin decomposition product according to claim 1, wherein the lignin comprises at least one selected from the group consisting of kraft lignin and lignin derivative.

3. The method for producing a lignin decomposition product according to claim 1 or 2, wherein the lignin comprises lignin extracted from bamboo.

4. The method for producing a lignin decomposition product according to claim 1 or 2, wherein the visible light includes a wavelength of 385 to 740 nm.

5. The method for producing a lignin decomposition product according to claim 1 or 2, wherein the decomposition step comprises irradiating the mixture with the visible light while stirring the mixture.

6. The method for producing a lignin decomposition product according to claim 1 or 2, wherein the lignin decomposition product contains methanol.

7. The method for producing a lignin decomposition product according to claim 1 or 2, wherein the lignin decomposition product contains a catechol structure represented by the following formula (1):

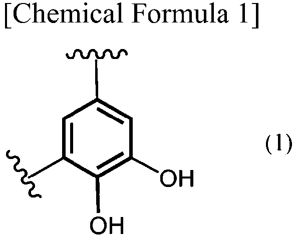

8. A method for producing methanol, the method comprising a step of generating methanol by irradiating, with visible light, a mixture comprising a substrate containing lignin and a catalyst containing at least one selected from the group consisting of divalent and trivalent metal ions.

9. A lignin-based composition comprising:
a lignin decomposition product obtained by decomposing lignin by irradiating, with visible light, a mixture comprising a substrate containing the lignin and a catalyst comprising at least one selected from the group consisting of divalent and trivalent metal ions; or
a purified product of the lignin decomposition product.

10. The lignin-based composition according to claim 9, wherein the lignin decomposition product contains a catechol structure represented by the following formula (1):
